# EUROPEAN PATENT APPLICATION

(11) **EP 4 755 322 A1**
(43) Date of publication of application: **10.06.2026**
(21) Application number: 24216977.9
(22) Date of filing: 03.12.2024
(51) Int. Cl.: A61B 17/062, A61B 34/30, A61B 34/00, A61B 90/00, A61B 17/00

(54) **END-EFFECTOR SYSTEM**

(71) Applicant: Microsure B.V., 5692 EA Son (NL)
(72) Inventor: Boerma, Erik Niels, 5616 BS Eindhoven (NL)
(74) Representative: DTS Patent- und Rechtsanwälte PartmbB

(57) **Abstract**

The present invention relates to an end-effector system for a surgical, microsurgical or super-microsurgical robot arm and a method of mapping a user input device (400) in an end-effector system.

## Description

The present invention belongs to the technical field of robotic systems and techniques for use in surgical, microsurgical or super-microsurgical procedures.

The invention provides an end-effector system, in particular for a surgical, microsurgical or super-microsurgical robot arm.

As a non-limiting example, the robot arm can be used to perform anastomoses.

Provided is also method of mapping a user input device in an end-effector system as defined above. Different kinds of surgical instruments are usually necessary when a surgical, microsurgical or super-microsurgical operation is carried out.

These surgical instruments are often configured to be manually held and manipulated by the operator.

Alternatively, said surgical instruments can be manipulated through a robotic system comprising one or more robot arms.

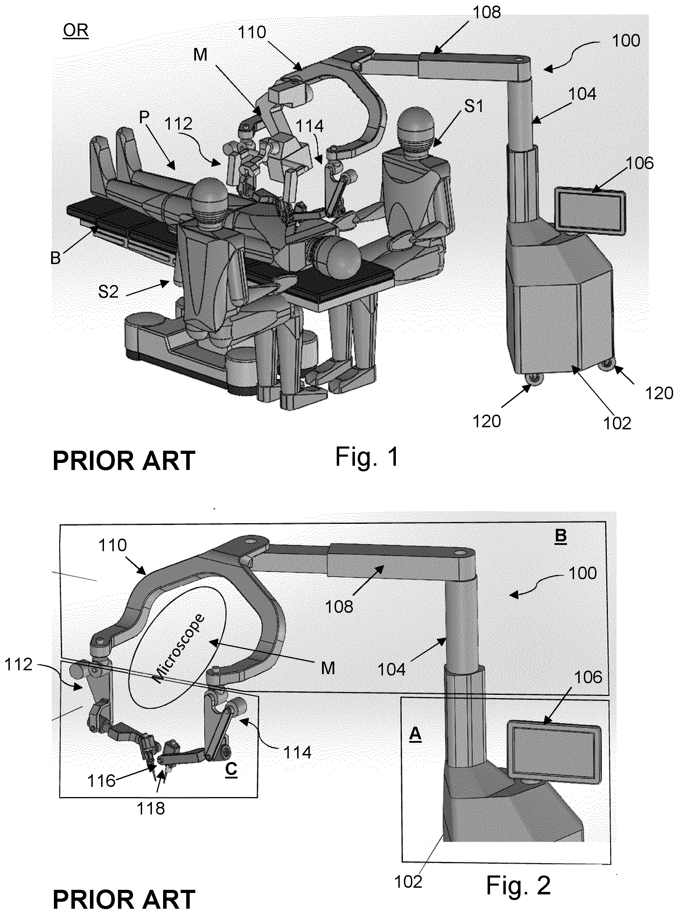

An exemplary configuration of a robotic system for use in surgery, microsurgery or super-microsurgery is shown in **Figs. 1-2****.**

The use of a robotic system facilitates accurate movements with micrometer precision without substantial tremor, thereby superseding the limitations of human hand manipulation.

Here, the use of an adapter is required to provide an interface between the surgical instrument and the end-effector of the robot arm.

Surgical instrument adapters for use in robotic systems of this kind are known in the art.

An exemplary surgical instrument adapter is described in WO2022233585A1, filed in the name of the same applicant **(****Fig. 3****).**

Here, the surgical instrument adapter comprises a clip to at least one surgical instrument, configured to hold the at least one surgical instrument in a predefined orientation with respect to the end-effector, and to transmit a movement of a tip portion of first and second fingers of the end-effector to the at least one surgical instrument, to manipulate said at least one surgical instrument.

Another surgical instrument adapter is disclosed in EP23207208, filed in the name of the same applicant.

Surgical, and in particular microsurgical or super-microsurgical procedures require precise and reliable manipulation of one or more surgical instruments connected to the end-effector of the robotic arm.

Hinged surgical instruments, such as scissors-type or forceps-type surgical instruments, are often used in surgical, microsurgical or super-microsurgical procedures.

These kinds of instruments are characterized by a proximal end and a distal end, the distal end comprising two functional distal portions, i.e., first and second arms, configured to perform a movement with respect to one another (e.g., open/close).

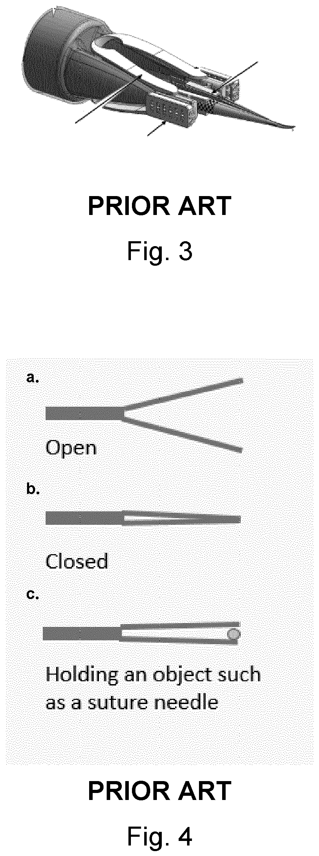

Surgical instruments of this kind are adapted to switch between an open state and a closed state, as schematically shown in **Fig. 4****.**

The instrument has a certain first stiffness in the open state and an second certain stiffness in the closed state (either closed without an object like a needle in between or gripping an element like a needle, resulting in both cases with the second certain stiffness, which is larger than the first certain stiffness), which has to be taken into account and is taken into account as part of the system and method according to the present disclosure/invention.

**Fig. 4a** shows an open state of the instrument; **Fig. 4b** shows a closed state of the instrument, where the first and second arms of the instrument are in contact with each other; **Fig. 4c** also shows a closed state of the instrument, but where an object, e.g., a suture needle, is grasped between the first and second arms of the instruments. Here, the closure point of the instrument is different compared to the case of **Fig. 4b** due to the presence of the instrument grasped between first and second arms.

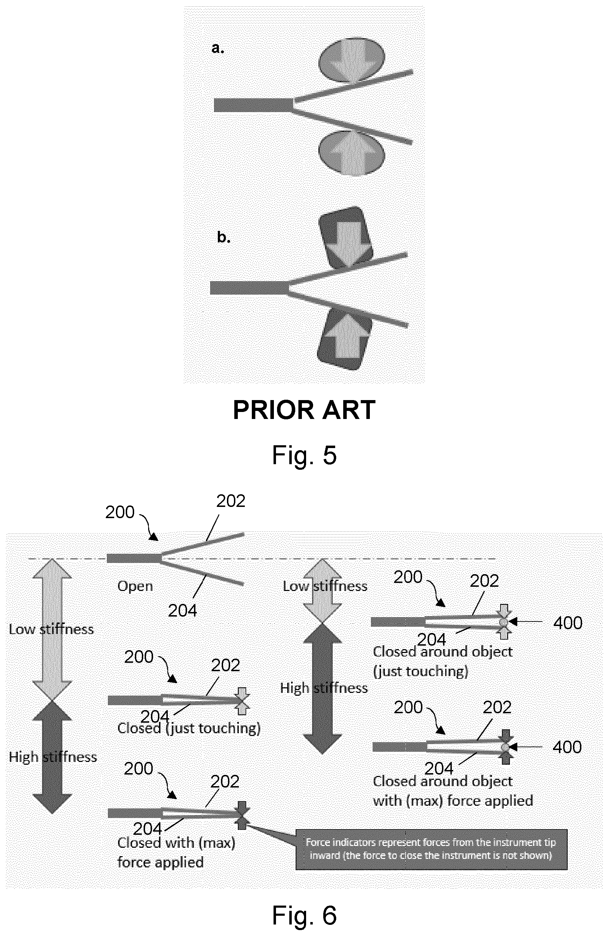

The surgical instrument can be transitioned from the open state to the closed state upon exerting a pressing force on a middle portion of the instrument through the user's fingers, as shown in **Fig. 5a.**

Alternatively, in case a surgical robot is used for manipulation of the instrument, the pressing force can be exerted through first and second fingers of an end-effector, as shown in **Fig. 5b.**

In scissors-type instruments, the two distal portions are configured to move in the same fashion as a pair of scissors.

Here, a hinge mechanism is provided in the proximity of said distal portions.

On the other hand, in forceps-type instruments, the two distal portions are adapted to move in the same fashion as tweezers.

Here, a hinge mechanism is rather provided at the proximal end of the instrument.

When a surgical instrument is manually manipulated, the user receives a direct haptic feedback corresponding to a current state/transition in state of the instrument, e.g., when a closure point of the instrument has been reached **(Figs. 4b-4c).**

In microsurgical and super-microsurgical procedures, surgical microscopes may also be used to provide a visual feedback to the user, to complement proprioception.

On the other hand, when the instrument is manipulated though a robotic system, the system may be unable to transmit any sense of force, effort, heaviness, or the like (i.e., a proprioceptive feedback) to the user. This is likely the case when using an input device having a simplified structure.

As discusses in El Rassi, I. et al. **[1]**, during traditional surgery, the surgeon's hands are in direct contact with organs, and the surgeon can rely on the sense of touch to perform surgery. In teleoperated robotic systems, however, all physical connections between the surgeon and both the robot and patient are absent. Thus, the surgeon must estimate the force exerted on organs based only on visual deformation of tissues he/she is pulling, pushing, gripping, or suturing. In a worst-case scenario, this may result in tissue injury and inability to perform complex manipulation.

The lack of a haptic feedback is problematic especially when operating on delicate soft tissues or using fragile materials, increasing the risk of damages.

When a surgical, microsurgical or super-microsurgical procedure is carried out, it is of the utmost importance for the operator to sense:
- whether tips of the first and second arms of the instrument are in contact with each other;
- whether the first and second arms are holding/manipulating an object, e.g., a surgical needle, suture wire, or a living tissue of a patient, e.g., a vessel;
- the amount of force applied to the instrument, and
- the amount of force applied when pushing or pulling an object (e.g., when pushing a needle into a target tissue such as a blood vessel, or when pulling a surgical wire to tie a knot).

Also, when microsurgical or super-microsurgical procedures are carried out, forces that are applied to target tissues, e.g., blood vessels, are significantly small.

This hinders the possibility of properly detecting the applied forces, in turn jeopardizing the capability of providing a reliable haptic feedback for the user.

In this regard, experiments carried out by the inventors revealed there is almost no difference in applied forces when a scissors-type surgical instrument is simply brought to a closed state, and when a cutting operation is performed (a poly-vinyl alcohol vessel model, mimicking real tissue and used in microsurgery training, was used for the experiment).

This means that, in practice, the surgeon is not capable to feel a proper difference between the two situations.

In the absence of a haptic feedback, it becomes difficult for the operator to clearly distinguish a current status of the instrument, and precisely tune the applied force (increase/decrease) to achieve a desired surgical outcome.

This is even more problematic in the context of microsurgical or super-microsurgical procedures, requiring a significantly high level of precision.

For instance, the user may need to decrease a gripping force exerted by the instrument on an object, e.g., a surgical needle, without said object being completely released.

Here, it is crucial for the surgeon to understand when to stop releasing the gripping force to prevent occurrence of grip loss, in turn resulting in the object being unwantedly released.

As an example, to perform piercing of a vessel, a surgical needle must be firmly held between the first and second arms of the surgical instrument.

However, when (re)positioning of the needle is required, the needle must be held with a lighter gripping force to allow for adjustment.

Accordingly, the gripping force exerted on the object shall be reduced.

Nevertheless, an excessive decrease in the exerted gripping force would result in complete releasing, causing the needle to drop.

Bergholz M. et al. **[2]** discusses the benefits of artificial haptic feedback in robot assisted surgery, e.g., in terms of reducing average forces and peak forces applied during surgery, reducing completion time, and enhancing accuracy and success rates during surgical tasks. So far, the provision of a haptic feedback for the surgeon in robotic surgery has proven difficult to achieve. In particular, technical limits in determining the parameters in combination with available volume and mass in both the end-effector and the input device, made the provision of a proper haptic feedback problematic.

Haptic sensors can be used to collect and send haptic information, that are displayed on the operator's side, creating telepresence (known as transparency). Multiple ways have been developed to improve transparency through force feedback and tactile feedback. However, this has the drawback of interfering with the stability of closed-loop controlling interactions between master, robot and remote environment **[1].**

Cutaneous feedback is more stable but less transparent, while force feedback is more transparent but less stable. Thus, multimodal platforms of haptic feedback would try to find the best trade-off between both modalities **[1].**

A partial solution is described in US20220361966A1, inter alia disclosing a solution where a haptic feedback is provided through a device configured to be held by the surgeon through his/her hands. For instance, said device may comprise a small servomotor connected to two members. Alternatively, the surgeon may be provided with an exoskeleton-like device to wear on each of his/her arms, allowing the surgeon to experience haptic feedback for each robotic actuator. Other standard haptic interaction devices may also be used. Haptic feedback is used to provide the user of a surgical robotic system with a physical sensation in order to convey certain useful or critical information to the driving of the robotic system.

However, this known solution still has some drawbacks.

In particular, the provision of a device to be held or worn by the user, such as an exoskeleton-like device to be worn on the arm, may be uncomfortable.

Similarly, the incorporation of a servomotor renders the device quite heavy, thus more burdensome to manipulate. This may also jeopardize reliability of the provided haptic feedback.

Also, this adds structural complexity to the robotic system, since an additional component, i.e., the haptic feedback device, must be provided and controlled. Still further, the provided haptic feedback may still not match the current requirements in terms of precision and reliability, especially in case of microsurgical or super-microsurgical procedures.

Hence, there is room for further improvement.

In the light of the above, it is an object of the present invention to provide an end-effector system for a surgical, microsurgical or super-microsurgical robot arm allowing an operator, e.g., a surgeon, to control operation of a robotically-manipulated hinged surgical instrument with improved ease, precision, and reliability.

According to the invention, an end-effector system for a surgical, microsurgical or super-microsurgical robot arm, at least comprises:
a hinged surgical instrument configured to switch between an open state and a closed state, said surgical instrument comprising a first arm and a second arm,
connected through a hinge;
an end-effector, configured to control operation of the surgical instrument;
an input device comprising a user interface configured to receive an input from a user, and
a controller,
wherein the controller is configured at least to:
define a first state of the surgical instrument, characterized by a first value of at least one predefined parameter, said first state corresponding to an open state of the instrument;
define a second state of the surgical instrument, characterized by a second value of said at least one predefined parameter, different from the first value, said second state corresponding to a closed state of the instrument;
define an original transition point between the first state and the second state of the surgical instrument based on a detected change in said at least one predefined parameter from the first value to the second value, and vice versa, and
map operation ranges for the input device based on the defined original transition point.

The present invention provides an end-effector system for a surgical, microsurgical or super-microsurgical robot arm.

As a non-limiting example, the robot arm can be used to perform anastomoses.

The system comprises a hinged surgical instrument.

In particular, the surgical instrument is configured to switch between an open state and a closed state.

The surgical instrument comprises a first arm and a second arm, connected through a hinge.

For instance, the surgical instrument can be a scissors-type or a forceps-type surgical instrument.

The system further comprises an end-effector.

In particular, the end effector is configured to control operation of the surgical instrument.

The system further comprises a user input device.

The user input device comprises a user interface, configured to receive an input from a user.

The system further comprises a controller.

The controller is configured to define a first state of the surgical instrument, characterized by a first value of at least one predefined parameter.

This first state corresponds to an open state of the surgical instrument.

The controller is further configured to define a second state of the surgical instrument, characterized by a second value of said at least one predefined parameter.

Said second value is different from the first value.

This second state corresponds to a closed state of the instrument.

The controller is further configured to define an original transition point between the first state and the second state of the surgical instrument.

In particular, the transition point is defined based on a detected change in said at least one predefined parameter from the first value to the second value, and vice versa.

The controller is further configured to map operation ranges for the input device based on the defined original transition point.

The invention is based on the basic idea that, by mapping operation ranges of a user input device based on a defined transition point between different states of a robotically-manipulated hinged surgical instrument, reliability and accuracy in operating the instrument can be significantly improved, this being beneficial to the overall surgical outcome. This even more applies in case of microsurgical or super-microsurgical procedures, requiring a significantly high level of precision. Also, the surgical instrument can be operated by the user with greater ease, even in the absence of a haptic feedback.

As a non-limiting example, the user input device can be in the shape of a joystick, allowing the user to easily and intuitively provide a user input through a joystick lever.

In particular, the second state of the surgical instrument can be a state where tip portions of the first and second arms of the surgical instrument contact each other, as shown in **Fig. 4b.**

Alternatively, said second state can be a state where the first and second arms 202, 204 of the surgical instrument hold an object therebetween, as shown in **Fig. 4c.**

When the object is grasped between the first and second arms of the surgical instrument, the transition point changes due to the volume of the object.

Upon detecting that the transition point has changed, the controller is further configured to define an updated transition point between the first state and the second state of the surgical instrument.

Then, the controller is configured to re-map operation ranges for the input device based on the defined updated transition point.

Accordingly, the risk of inaccurate manipulation of the surgical instrument due to mismatch-related errors can be largely prevented.

Otherwise stated, re-mapping ensures that operation ranges for the input device are based on the current state/transition point of the surgical instrument.

These newly-defined operation ranges for the input device remain valid until a further change in the transition point occurs, e.g., when the object is released.

When the object is released, the controller is configured to determine that the transition point between the first state and the second state of the surgical instrument is back to the original transition point.

Then, the controller is further configured to re-map operation ranges for the input device based on the original transition point.

That is, operation ranges for the input device are reverted to the original state.

In one example, said object can be a surgical needle.

In another example, said object can be a suture wire.

In a still further example, said object can be a living tissue of a patient, in particular a blood vessel.

In general, said object can be any kind of object subject to manipulation during a surgical, micro-surgical or super-microsurgical operation, such as fixations that enable better access to a task at hand, e.g., artery clips, spreaders, or plastic strips for visual contrast.

Advantageously, the controller is configured to actuate the end-effector to transition the surgical instrument from the first state to the second state upon detecting that a force exerted by the user on the user interface of the input device is above a threshold value corresponding to the transition point.

Similarly, the controller is configured to actuate the end-effector to transition the surgical instrument from the second state to the first state upon detecting that a force exerted by the user on the user interface of the input device is below the threshold value corresponding to the transition point.

That is, until the exerted force is not above or falls below the predefined threshold value, transition from the first state to the second state, or vice versa, does not occur.

For instance, when a force exerted by the user on the user interface is decreased while an object is grasped between the first and second arms of the instrument, the object is not completely released until said force falls below a predefined threshold value.

This is advantageous in case it is required to slightly release the gripping force, e.g., for object repositioning, but without determining complete releasing of the object.

One potential issue is that no haptic feedback is provided for the user when the surgical instrument is transitioned/about to be transitioned from the first state to the second state, and vice versa.

Otherwise stated, while operating the input device to manipulate the instrument, the user always perceives the same stiffness, so that transition of the instrument between the first and second states is not clearly detectable through a prompt tactile feedback.

This may be problematic, especially when operating closely around the transition point.

To obviate the problem, the input device may have at least a first stiffness condition and a second stiffness condition.

The first stiffness condition is characterized by a first stiffness value.

The second stiffness condition is characterized by a second stiffness value.

In particular, the second stiffness value is higher than the first stiffness value.

The change in stiffness occurring when the input device is transitioned from the first stiffness condition to the second stiffness condition, and vice versa, provides a haptic feedback that is sensed by user through the user interface.

In one configuration, the transition point between the first stiffness condition and the second stiffness condition of the input device corresponds to the transition point between the first state and the second state of the surgical instrument.

That is, transition between the first stiffness condition and the second stiffness condition of the input device, and transition between the first state and the second state of the surgical instrument occur simultaneously.

Accordingly, when a change in stiffness is sensed by the user through the user interface, the user is aware that a corresponding change in state for the surgical instrument occurred.

For instance, upon sensing an increase in stiffness, denoting that a transition from the first stiffness condition to the second stiffness condition occurred, the user becomes aware that an object, e.g., a surgical needle or suture, is reliably grasped between the first and second arms of the surgical instrument.

Also, it may be the case that the grasping force exerted on the object through the first and second arms of the instrument shall be released slightly to allow for (re)positioning of the object (without complete release).

In such a case, the user is aware that, as long as the sensed stiffness remains unchanged, the surgical instrument is still grasped by the instrument.

Alternatively, the transition point between the first stiffness condition and the second stiffness condition of the input device can be offset with respect to the transition point between the first state and the second state of the surgical instrument.

Preferably, the transition point between the first stiffness condition and the second stiffness condition of the input device is prior in time with respect to the transition point between the first state and the second state of the surgical instrument.

This solution is advantageous because the user is enabled to feel an upcoming state transition for the instrument just before it occurs.

For example, this may be useful to control opening of the instrument to slightly release the gripping force exerted on an object by the first and second arm, without completely releasing the object.

Moreover, this may be useful in manipulating a surgical needle before piercing a vessel.

Also, abrupt opening of the tip portions of a dilator may lead to tissue damage. By feeling the transition in the input device just before it occurs in the instrument, this can be largely prevented.

The first stiffness condition of the input device can be determined by a first elastic means.

In particular, said first elastic means is actuated over an entire opening/closing range of the surgical instrument.

The second stiffness condition of the input device can be determined by a second elastic means.

In particular, said second elastic means is configured to provide additional stiffness upon being operated.

The change in stiffness generated by the operation/deactivation of the second spring is sensed by the user through the user interface of the input device, thus providing a haptic feedback denoting that a change in state for the surgical instrument occurred/is about to occur.

Preferably, said first and second elastic means is a spring.

Conveniently, the input device may have at least one intermediate stiffness condition between the first stiffness condition and the second stiffness condition.

The intermediate stiffness condition is characterized by an intermediate stiffness value.

In particular, this intermediate stiffness value is comprised between the first stiffness value and the second stiffness value.

Advantageously, the intermediate stiffness condition can be determined by an additional elastic means.

Similar as above, the additional elastic means is preferably a spring.

Here, instead of a single transition, multiple, e.g., two, transitions occur in the input device, providing a corresponding haptic feedback for the user.

This can be used for operation around the closure point of the instrument.

For instance, softly gripping of an object, e.g., a needle, can be simulated.

Also, in case of a dilator, this can help predicting when the instrument will open.

Still further, in case of a needle holder, this can help indicating the range of closure in which the needle is held loosely.

In the intermediate stiffness condition, operation ranges for the input device may be re-mapped or may not re-mapped.

Advantageously, the system may further comprise a sensor means.

In particular, said sensor means is configured to detect a change for said at least one predefined parameter from the first value to the second value, and vice versa.

Preferably, the at least one predefined parameter includes one or more among stiffness, coordinate points for the instrument tip, and a position and/or force at the instrument tip.

For instance, a sensor value of 100% can be set for an open state of the instrument, while a sensor value of 0% can be set for a closed state of the instrument, when tip portions of the first and second arms contact each other.

A sensor value of X% can be set for an expected closure point of the instrument around an object, e.g., a surgical needle or suture.

Operation ranges for the input device are mapped accordingly.

As a non-limiting example, said sensor means can be arranged both in the input device and the end-effector, to allow for mapping. Here, the end-effector may be provided, e.g., with a force sensor and a position sensor.

In general, the end-effector may comprise an end-effector base, a first movable finger, and a second movable finger.

The first and second movable fingers extend distally from the end effector base.

Also, the first and second movable fingers are capable of performing an opening/closing movement with respect to each another.

In detail, the opening/closing movement of the first and second fingers can be obtained by the provision of a hinge located between the fingers and a base portion of the instrument, allowing for movement of both fingers with the same, inverted magnitude.

The end-effector may further comprise a surgical instrument adapter.

In particular, the surgical instrument adapter is configured to transmit a movement of a tip portion of the first and second movable fingers of the end-effector to the surgical instrument.

Also, the system may include a sterile drape, configured to cover the end-effector.

This allows maintaining the sterile barrier.

The invention further provides a method of mapping a user input device in an end-effector system as defined in the foregoing.

The method at least comprises:
defining a first state of the surgical instrument, characterized by a first value of at least one predefined parameter, said first state corresponding to an open state of the instrument;
defining a second state of the surgical instrument, characterized by a second value of said at least one predefined parameter, different from the first value, said second state corresponding to a closed state of the instrument;
defining an original transition point between the first state and the second state of the surgical instrument, based on a detected change in said at least one predefined parameter from the first value to the second value, and vice versa, and
mapping operation ranges for the input device based on the defined original transition point.

The second state of the surgical instrument can be a state where tip portions of the first and second arms of the surgical instrument contact each other.

Alternatively, the second state can be a state where the first and second arms of the surgical instrument hold an object therebetween.

In this case, the method may further comprise:
when the object is grasped between the first and second arms of the surgical instrument, determining that the transition point between the first state and the second state of the surgical instrument has changed;
defining an updated transition point between the first state and the second state of the surgical instrument, and
re-mapping operation ranges for the input device based on the defined updated transition point.

Also, the method may further comprise:
when the object is released, determining that the transition point between the first state and the second state of the surgical instrument is back to the original transition point, and
re-mapping operation ranges for the input device based on the original transition point between the first state and the second state.

In general, the method can be implemented through a computer-readable non-volatile storage medium comprising computer-readable instructions that, when executed by a processing means, causes said processing means to implement and/or process and/or perform the method steps described in the foregoing.

Further advantages of the present invention shall now be disclosed in connection with the drawings, where:
- **Fig. 1**: shows a robotic system for use in surgery, microsurgery or super-microsurgery according to the prior art, in an operative state;
- **Fig. 2**: shows a detail of the surgical robotic system of **Fig. 1****,** where different sections of the robotic system, respectively denoted with letters **A, B, C,** are identified;
- **Fig. 3**: shows an end-effector system provided with a surgical instrument adapter according to the prior art, in particular as described in WO2022233585A1. Here, a surgical instrument is mounted to the adapter;
- **Fig. 4**: is a diagram showing different states of a hinged surgical instrument, namely: **a.** open state, **b.** closed state, and c. closed state holding an object, here a suture needle, between first and second arms of the surgical instrument;
- **Fig. 5**: is a diagram showing how first and second arms of a hinged surgical instrument can be brought from an open state to a closed state, namely **a.** by the fingers of an operator exerting a pressing force on a middle portion of the instrument, **b.** by first and second fingers of an end-effector of a robot arm exerting a pressing force on a middle portion of the instrument;
- **Fig. 6**: is a diagram showing stiffness characterizing different states of a hinged surgical instrument (namely: open, closed, and closed holding an object, as illustrated in **Fig. 4**);
- **Fig. 7**: is a diagram showing different states of an input device, e.g., a joystick, for corresponding states of the surgical instrument (as illustrated **Fig. 4**). Here, a sensor means is provided for detecting a current state or a transition in state of the surgical instrument, based on a current value or a change in value for at least one predefined parameter;
- **Fig. 8**: is a diagram providing an overview of the operation of the input device, e.g., a joystick, and the hinged surgical instrument, for different states of the surgical instrument (as illustrated **Fig. 4****);**

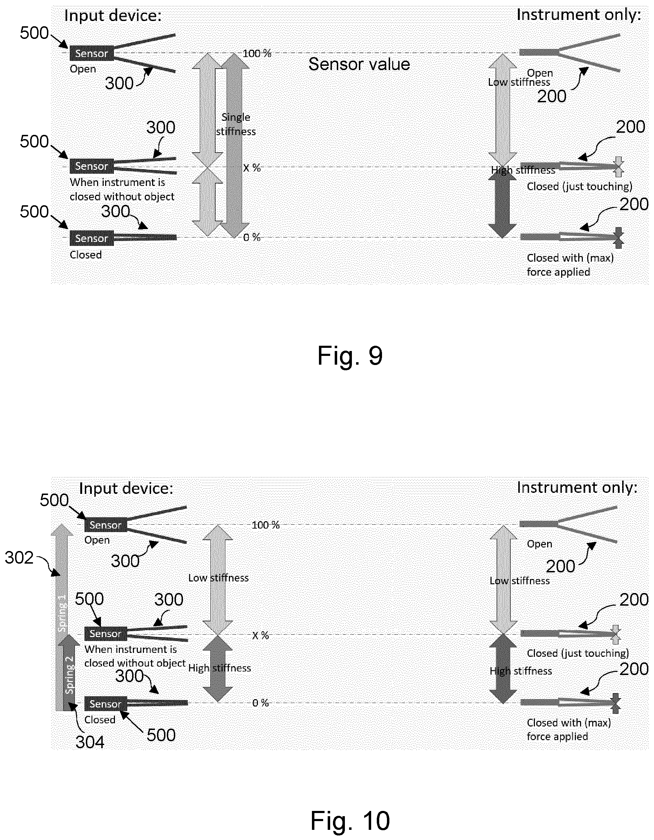

- **Fig. 9**: is a diagram showing mapping of the input device based on different states of the surgical instrument. Here, a sensor value of 100% corresponds to an open state **(Fig. 4a),** a sensor value of 0% corresponds to a closed state where tip portions of the first and second arm of the instrument contact each other **(Fig.4b),** while a sensor value of X% corresponds to an expected closure point around an object **(Fig. 4c**). Operation of the input device is mapped accordingly. Here, different states of the instrument are characterized by a respective level of stiffness. Conversely, a stiffness condition of the input device remains the same for all states of the surgical instrument;
- **Fig. 10**: is a diagram similar to that of **Fig. 9****,** but where the input device is characterized by a first stiffness condition and a second stiffness condition. Here, the transition point between the first stiffness condition and the second stiffness condition corresponds to the transition point between the first state and the second state of the surgical instrument. The first stiffness condition is determined by a first spring (Spring 1), actuated over an entire opening/closing range of the surgical instrument, while the second stiffness condition is determined by a second spring (Spring 2), configured to provide additional stiffness upon being operated.
- **Fig. 11**: is a diagram showing a configuration similar to that of **Fig. 10****,** but where the transition point between the first stiffness condition and the second stiffness condition is offset with respect to the transition point between the first state and the second state of the surgical instrument. Here, transition from the first stiffness condition to the second stiffness condition, and vice versa, occurs prior in time with respect to the transition from the first state to the second state of the surgical instrument, and vice versa;
- **Fig. 12**: is a diagram showing a solution similar to that of **Fig. 10****,** but where an additional spring (Spring 3) is provided to determine an intermediate stiffness condition between the first stiffness condition and the second stiffness condition, characterized by an intermediate stiffness value.
- **Fig. 13**: is a diagram similar to that of **Fig. 10****,** in particular emphasizing that, when an object is grasped between the first and second arms of the surgical instrument, the transition point/closure point of the instrument is shifted compared to the case of an empty instrument. This may cause mismatch-related errors in case operation ranges for the input device are not re-mapped accordingly;
- **Fig. 14**: is a diagram showing how characteristics of the closure point of the hinged surgical instrument are determined;
- **Fig. 15**: is a diagram showing how the transition point between the first and second states of the instrument is shifted when an object, e.g., a surgical needle or suture, is grasped between the first and second instrument arms;
- **Figs. 16-19**: are diagrams schematically illustrating mapping and dynamic re-mapping of operation ranges of the input device for different states of the surgical instrument.

**Fig. 1** shows an example of a robotic system for use in surgery, microsurgery or super-microsurgery.

Said exemplary system is denoted with 100.

Here, the system 100 is shown in an operating state.

A first surgeon S1 and a second surgeon S2 are located at a respective side of an operating bed B to perform a surgical, microsurgical or super-microsurgical operation on a patient P, laying on operating bed B.

The robotic system 100 includes a base station 102.

The surgical robotic system 100 further includes a base column 104, here in the shape of a cylindrical pillar.

In the shown example, the base station 102 conveniently includes a display module 106 for displaying information to one or more operators, e.g. the surgeons S1, S2 or other personnel that is present in the operating room OR.

Also, the display module 106 may be used to define a user interface allowing one or more operators to provide a user input.

The base station 102 comprises a plurality of wheels 120 for ease of placement of the robotic system 100 at a desired location within the operating room OR.

In the shown example, the robotic system 100 is equipped with a suspension arm 108, a fork-like element 110, a first surgical, microsurgical or super-microsurgical robot arm 112, and a second surgical, microsurgical or super-microsurgical robot arm 114.

Here, the suspension arm 108 is horizontally arranged.

Also, the suspension arm 108 has two parts allowing to adjust its length by telescopically moving said parts relatively to each other.

It is also possible that a different means is used for length adjustment of the suspension arm 108, e.g. a SCARA-mechanism, hinge mechanisms, or the like.

The suspension arm 108 is connected to the base column 104.

The suspension arm 108 carries the fork-like element 110 (e.g. a bracket).

As shown in detail in **Fig. 2****,** the fork-like element 110 carries the first surgical, microsurgical or super-microsurgical robot arm 112 and the second surgical, microsurgical or super-microsurgical robot arm 114.

Each of the first and second robot arms 112, 114 is connected to an end-effector system carrying a surgical instrument 116, 118 **(****Fig. 2****).**

The surgical instruments 116, 118 may be of the same kind, or may be different from each other.

The suspension arm 108 is configured to rotate about the longitudinal axis of the base column 104, to allow desired positioning of the fork-like element 110 and the robot arms 112, 114 with respect to the surgical site.

Similarly, the fork-like element 110 is configured to rotate about an axis of the suspension arm 108, i.e. parallel to the longitudinal axis of the base column 104.

The robotic system 100 may be adapted for use with a conventional microscope M, as shown in **Fig. 2****.**

Alternatively, a fully digital microscope (not shown) or a so-called hybrid microscope (not shown) can be used. In such a case, one or more surgeons will be located at a separate console, and a robot trolley will be located at the position of one of the surgeons S1, S2 shown in **Fig. 1****.**

The surgical robotic system 100 provides a large patient side setup, this meaning that no assembly is required after draping.

**Fig. 2** shows different sections A, B, C of the surgical robotic system 100.

In section A, there is no movement and everything is thus stationary.

The display means 106 (included in section A) can be activated by one or more operators.

In section B, there is some movement during the surgery, in order to correctly reposition the surgical instruments 116, 118 over the patient P.

As shown in **Fig. 2****,** a space is defined between the surgical robotic arms 112, 114 for a microscope (not illustrated but only symbolized in **Fig. 2**).

Section C includes, inter alia, the end-effector.

Here, several movements occur in the course of the surgical procedure.

In this context, the functions of the end-effector are, inter alia, to actuate the surgical instruments 116, 118, provide a mounting interface, and rotate around the instrument axis.

In the following, a system for a surgical, microsurgical or super-microsurgical robot arm according to an embodiment of the invention will be described in detail.

As a non-limiting example, the robot arm can be used to perform anastomoses. The system comprises a hinged surgical instrument 200.

The surgical instrument 200 comprises a first arm 202 and a second arm 204, connected through a hinge, the first and second arms 202, 204 being capable of performing an open/close movement with respect to one another.

For instance, the surgical instrument 200 can be a scissors-type or a forceps-type surgical instrument 200.

The instrument 200 is configured to switch between an open state and a closed state, as schematically illustrated in **Fig. 4****.**

In the open state **(Fig. 4a),** the first and second arms 202, 204 of the instrument 200 can be spaced apart by a distance of 3 mm at the tips.

The closed state (second state) of the surgical instrument 200 can be a state where tip portions of the first and second arms 202, 204 contact each other, as schematically illustrated in **Fig. 4b.**

Alternatively, the closed state of the surgical instrument200 can be a state where the first and second arms 202, 204 of the surgical instrument 200 hold an object 400 therebetween, as schematically illustrated in **Fig. 4c.**

For instance, said object 400 can be a surgical needle.

Alternatively, said object 400 can be a suture wire.

As a further alternative, said object 400 can be a living tissue of a patient, in particular a blood vessel.

In general, said object 400 can be any kind of object subject to manipulation during a surgical, micro-surgical or super-microsurgical operation, such as fixations that enable better access to a task at hand, e.g., artery clips, spreaders, or plastic strips for visual contrast.

The system further comprises an end effector (not shown).

In particular, the end-effector is configured to control operation of the surgical instrument 200.

For example, the surgical instrument 200 can be transitioned from the first state to the second state by exerting a pressing force on a middle portion of the instrument 200 by first and second fingers of the end-effector, as schematically illustrated in **Fig. 5b.**

The system further comprises a user input device 300.

The user input device 300 comprises a user interface (not shown) configured to receive an input from a user.

For instance, the input device 300 can be in the shape of a joystick, allowing the user to provide a user input through a joystick lever.

The system further comprises a controller (not shown).

The controller is configured to define a first state of the surgical instrument 200, characterized by a first value of at least one predefined parameter.

The first state corresponds to the open state of the instrument 200 **(Fig. 4a).**

The controller is further configured to define a second state of the surgical instrument 200, characterized by a second value of said at least one predefined parameter.

The second value is different from the first value.

The second state corresponds to a closed state of the instrument 200.

As mentioned, the second state can be either a state where tip portions of the first and second arms 202, 204 of the surgical instrument 200 contact each other **(Fig. 4b),** or a state where an object 400 is grasped between the first and second arms 202, 204 **(Fig. 4c).**

The controller is further configured to define an original transition point between the first state and the second state of the surgical instrument 200, based on a detected change in said at least one predefined parameter from the first value to the second value, and vice versa.

For instance, the transition point may represent the point when the instrument 200 is transitioned from an open state **(Fig. 4a).**to a state where tip portions of the first and second arms 202, 204 contact each other **(Fig. 4b),**

The controller is further configured to map operation ranges for the input device 300, based on the defined original transition point.

As schematically shown in **Fig. 6****,** the open state of the instrument 200 is characterized by a first level of stiffness, while the closed state of the instrument 200 is characterized by a second level of stiffness, higher that the first level of stiffness. The highest level of stiffness is reached when the first and second arms 202, 204 reach their maximum closure point (either when contacting each other or when holding an object 400 therebetween).

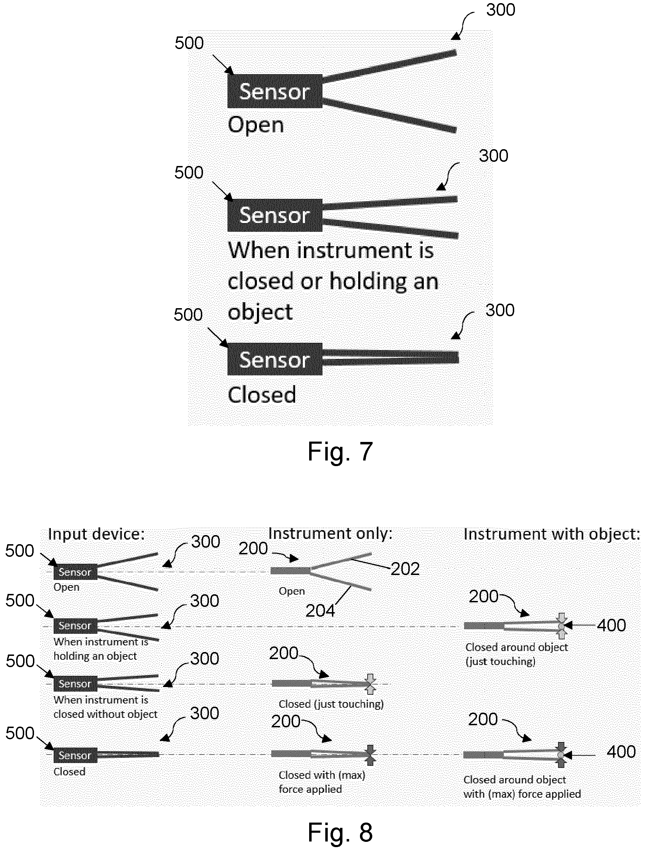

The system may further comprise a sensor means 500 configured to detect a change for said at least one predefined parameter from the first value to the second value, and vice versa **(****Figs. 7-8****).**

The at least one predefined parameter may include one or more among stiffness, coordinate points for the instrument tip, and a position and/or force at the instrument tip.

As a non-limiting example, said sensor means 500 can be arranged both in the input device 300 and the end-effector, to allow for mapping. Here, the end-effector may be provided, e.g., with a force sensor and a position sensor.

For example, as shown in **Fig. 9****,** a sensor value of 100% is set for an open state of the instrument 200 **(Fig. 4a),** while a sensor value of 0% is set for a completely closed state of the instrument 200, where tip portions of the instrument contact each other, as in **Fig. 4b.**

Also, a sensor value of X% is set for an expected closure point of the instrument 200 around an object 400 **(Fig. 4c),** e.g., a surgical needle or suture.

Operation ranges for the input device 300 are mapped accordingly.

For instance, as illustrated in **Fig. 9****,** the input device 300 can be mapped based on a detected sensor value as follows:
- 100%: fully open instrument;
- 0%: fully closed instrument (tips of the first and second arms contacting each other);
- X%: expected closure point around an object;
- Between X% and 100%: moving use of the instrument actuation;
- Between 0% and X%: clamping use of the instrument actuation.

One potential issue is that no haptic feedback is provided for the user when the surgical instrument 200 is transitioned from the first state and the second state, and vice versa.

As illustrated in **Fig. 9****,** the first and second states of the instrument 200 are characterized by a respective level of stiffness (lower for the open instrument 200 and higher for the closed instrument 200). However, a stiffness condition of the input device 300 remains unchanged for all states of the surgical instrument 200.

That is, the user feels the same stiffness regardless of the current state/transition in state of the instrument 200.

Hence, transition between the first and second states of the instrument 200 is not clearly detectable.

This may be problematic, especially when operating closely around the transition point.

To obviate this drawback, in the present embodiment, the input device 300 has at least a first stiffness condition characterized by a first stiffness value, and a second stiffness condition characterized by a second stiffness value, higher than the first stiffness value **(****Fig. 10****).**

In particular, in the configuration shown in **Fig. 10****,** the transition point between the first stiffness condition and the second stiffness condition of the input device 300 corresponds to the transition point between the first state and the second state of the surgical instrument 200.

Otherwise stated, transition between the first stiffness condition and the second stiffness condition of the input device 300, and transition between the first state and the second state of the surgical instrument 200 occur simultaneously.

Accordingly, when a change in stiffness is sensed by the user through the user interface, the user is aware that a corresponding change in state for the surgical instrument 200 occurred.

In some circumstances, this solution may still be sub-optimal, in particular because the user is enabled to feel the state transition only in the very moment in which it occurs, with no prior warning.

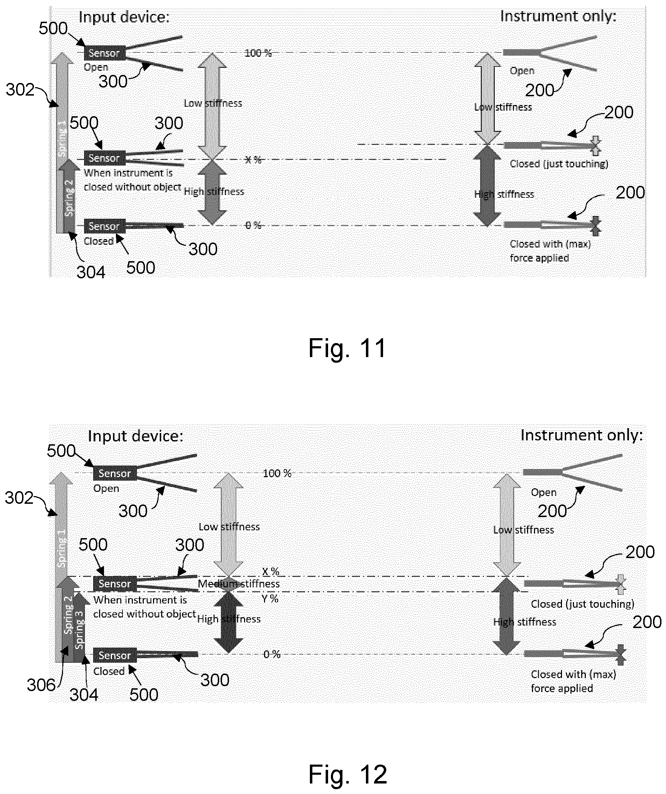

An improved alternative, allowing obviating the issue, is shown in **Fig. 11****.**

Here, the transition point between the first stiffness condition and the second stiffness condition of the input device 300 is offset with respect to the transition point between the first state and the second state of the surgical instrument 200.

The input device 300 is mapped accordingly.

In particular, in the present embodiment, the transition point between the first stiffness condition and the second stiffness condition of the input device 300 is prior in time with respect to the transition point between the first state and the second state of the surgical instrument 200.

Accordingly, when opening or closing the instrument 200, the user is enabled to feel the upcoming state transition just before it occurs in the instrument 200.

For example, this may be useful to control opening of the instrument 200 to slightly release the gripping force exerted on an object 400 by the first and second arms 202, 204, without completely releasing the object 400.

Moreover, this may be useful in manipulating a surgical needle before piercing a vessel.

Also, abrupt opening of the tip portions of a dilator may lead to tissue damage. By feeling the transition in the input device 300 just before it occurs in the instrument 200, this can be largely prevented.

In the present embodiment, the first stiffness condition of the input device 300 is determined by a first elastic means 302, here a spring, which is actuated over an entire opening/closing range of the surgical instrument 200 **(****Fig. 11****).**

On the other hand, the second stiffness condition of the input device is determined by a second elastic means 304, here a spring, configured to provide additional stiffness upon being operated **(****Fig. 11****).**

The change in stiffness generated by the operation/deactivation of the second spring 304 is sensed by the user through the user interface of the input device 300, thus providing a haptic feedback denoting that a change in state for the surgical instrument 200 occurred/is about to occur.

Advantageously, the input device 300 may also have at least one intermediate stiffness condition between the first stiffness condition and the second stiffness condition, characterized by an intermediate stiffness value.

In the present embodiment, said intermediate stiffness condition of the input device is determined by an additional elastic means 306, here a spring.

Here, instead of a single transition, multiple, e.g., two, transitions occur in the input device 300, providing a corresponding haptic feedback for the user.

For example, two adjacent transitions may be defined at sensor values X% and Y%, as shown in **Fig. 12****.**

This intermediate range can be used for operation around the closure point of the instrument 200.

For instance, softly gripping of an object, e.g., a needle, can be simulated.

Also, in case of a dilator, this can help predicting when the instrument will open.

Still further, in case of a needle holder, this can help indicating the range of closure in which the needle is held loosely.

In the intermediate stiffness condition, operation ranges for the input device 300 are not re-mapped.

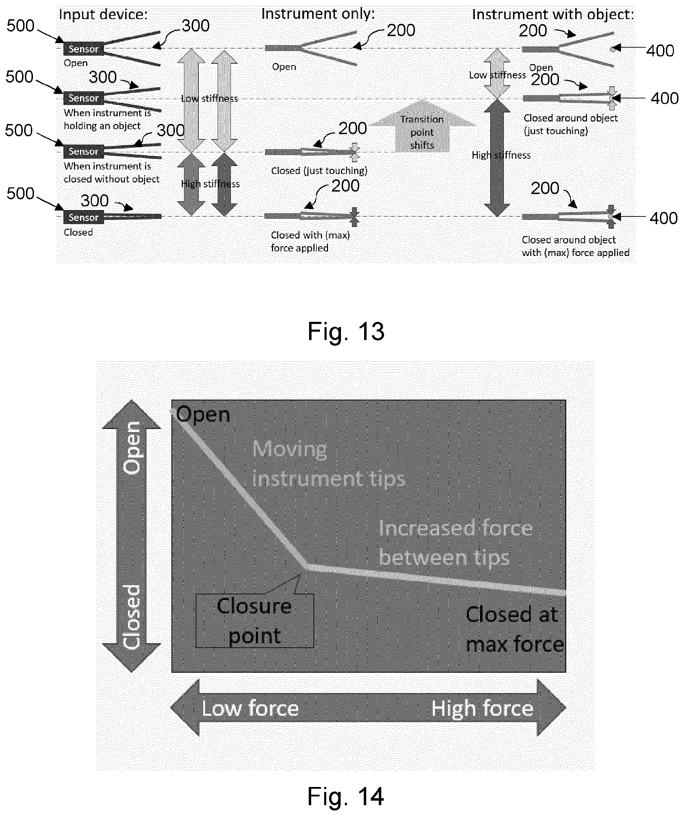

As it might be appreciated from **Figs. 6** and **13****,** when an object 400, e.g., a surgical needle or suture, is grasped between the first and second arms 202, 204 of the instrument 200, the transition point between the first and second states of the instrument 200 changes with respect to an original transition point defined for the empty instrument 200 (i.e., where the closed state is a state where tip portions of the first and second arms 202, 204 contact each other).

For example, when the instrument 200 is closed to grasp an object 400, the closure point (corresponding to an increase in stiffness) is reached at a different point compared to the case of an empty instrument 200.

In the present embodiment, when an object 400 is grasped between the first and second arms 202, 204 of the surgical instrument 200, the controller is configured to determine that the transition point between the first state and the second state of the surgical instrument 200 has changed.

The controller is further configured to define an updated transition point between the first state and the second state of the surgical instrument 200.

Also, the controller is configured to re-map operation ranges for the input device 300 based on the defined updated transition point.

Accordingly, the risk of inaccurate manipulation of the surgical instrument 200 due to mismatch-related errors can be largely prevented.

Otherwise stated, re-mapping of the operation ranges ensures that operation ranges for the input device 300 are based on the current state/transition point of the surgical instrument 200.

These newly-defined operation ranges for the input device 300 remain valid until a further change in the transition point occurs, e.g., when the object 400 is released.

When the object is released, the controller is configured to determine that the transition point between the first state and the second state of the surgical instrument 200 is back to the original transition point.

Then, the controller is further configured to re-map operation ranges for the input device 300 based on the original transition point.

That is, the input device 300 is re-mapped to its original operation ranges.

**Fig. 14** illustrates how characteristics of the closure point of the instrument 200 are determined according to the invention.

First, the instrument 200 is opened and closed, and both the force applied to the instrument 200 and the position of tip portions of end-effector fingers are recorded.

These values can also be replaced by representative factors, such as end-effector motor current.

As can be seen, from the state "Open" to the closure point, there is a linear increase of the force. At the closure point, the force increase continues, but there is almost no movement between the instrument tips, i.e. there is a change of inclination of the force curve (force for closure vs. movement)

So, the transition point between the open state and the closed state can be and is mathematically determined.

Experiments carried out by the inventors revealed that surgical instruments show near linear behavior in both the first state and the second state.

Eventually, characteristic parameters for the closure point, e.g., coordinates of the closure point, and/or gradient of both ranges, are determined.

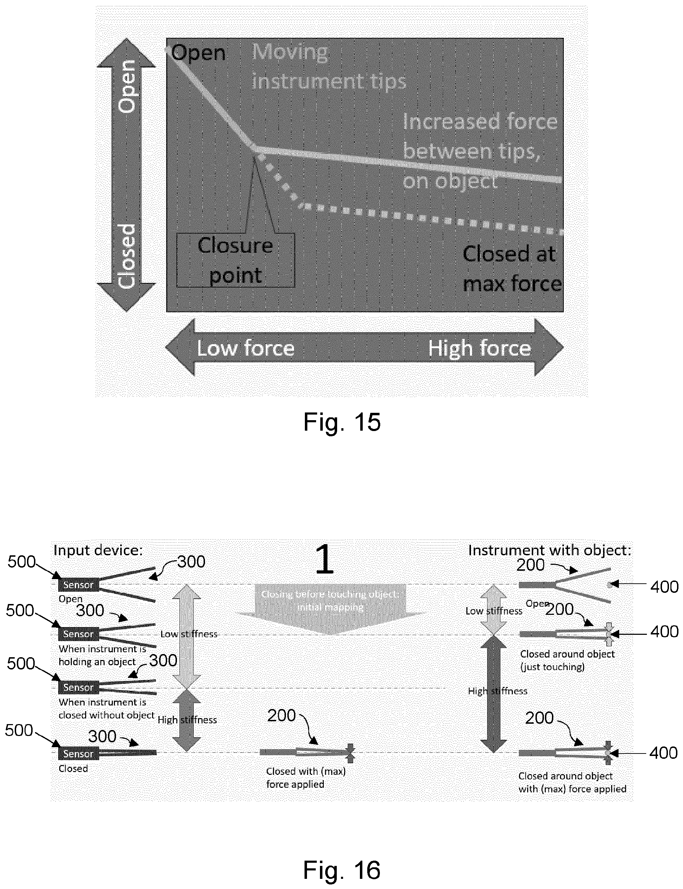

**Figs. 15** illustrates how the transition point between the first and second states of the instrument 200 is shifted when an object 400, e.g., a surgical needle or suture, is grasped between the first and second instrument arms 202, 204.

The object 400 is contacted at different position and operating force compared to the original transition point defined for the empty instrument 200.

When grasping an object characterized by a certain level of stiffness, e.g., a surgical needle, stiffness of the instrument 200 is very similar to its stiffness when closed beyond its closure point without grasping the object 400.

When tip portion of the instrument 200 contact each other, a further increase in the exerted force causes the instrument tips and the remainder of the instrument 200 to bend. This condition is characterized by a certain level of stiffness. In case an object having a certain level of stiffness (e.g., a surgical needle) is grasped, there is a significant increase in stiffness in the compression direction. When stiffness is determined, the lowest stiffness is experienced. This leads to the same stiffness value in both situations.

This does not apply in case a soft object, e.g., a vessel, is grasped.

Here, object stiffness is hardly perceivable, and does not determine any sensor response.

However, when an object 400 is grasped between the first and second arms 202, 204, the closure point and the maximum closure point of the instrument 200 (corresponding to a maximum force exerted by the user through the input device 300) are reached at different positions compared to a case where the tip portions of the instrument 200 are brought into contact with each other, with no object 400 grasped therebetween.

This requires re-mapping of operation ranges of the input device 300, to prevent mismatch-related inaccurateness.

Then, when the object 400 is released, the input device 300 is re-mapped to its original operation ranges, following its initial curve.

**Figs. 16-19** schematically illustrate mapping and dynamic re-mapping of operation ranges of the input device 300 for different states of the surgical instrument 200.

As shown in **Fig. 16****,** the starting situation is an open input device 300 and an open surgical instrument 200.

The instrument 200 is placed around an object 400, e.g., a surgical needle or suture.

The input device 300 is slowly closed, and the instrument 200 follows along the initial mapping.

The object 400 is touched by both tip portions of the instrument first and second arms 202, 204.

Then, the operator slowly closes the input device 300 further to increase the grip on the object 400.

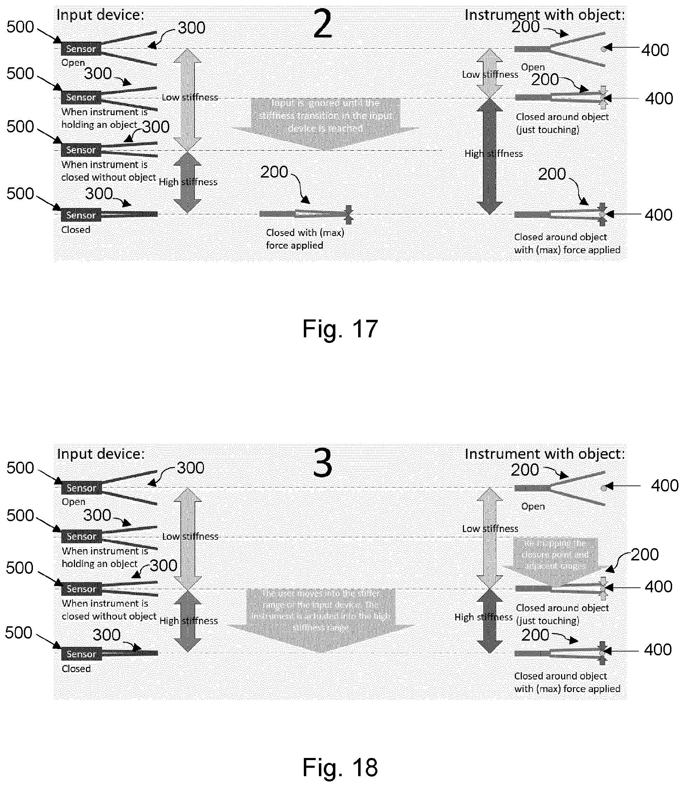

Upon contacting the object 400, the stiffness of the instrument 200 increases, as shown in **Fig. 17****.**

The increased closure demanded by the user leads to a different response in force increase than expected.

Active monitoring of predefined key parameters indicates a transition occurred in the instrument 200.

Then, corresponding re-mapping of the input device 300 is performed.

When the user operates the input device 300, e.g., by exerting a force on the user interface, the instrument 200 is kept static until a predefined threshold corresponding to the transition point is reached.

Then, upon reaching the higher stiffness of the input device 300, as shown in **Fig. 18****,** an input is fed from the input device 300 to the end-effector to further increase the grip on the object 400 as the exerted force is increased further.

As long as both the input device 300 and the instrument 200 remain in their stiffness states, opening and closing remains connected.

The input device 300, e.g., a joystick, shall always remain in a state corresponding to the actual state of the instrument 200.

For example, if the instrument 200 is in the open state, the input device 300 shall be in a corresponding open state. Analogously, if the instrument 200 is in the closed state, the input device 300 shall be in a corresponding closed state.

In each state, movement of the input device 300 is possible to control movement/closure force of the end-effector (and, in turn, of the surgical instrument 200).

That is, as long as the user keeps the input device 300 in one state, the end-effector (and, in turn, the surgical instrument 200) is maintained in the same state.

Then, when a state change occurs (e.g., because grip on an object (e.g., surgical a needle) is lost, re-mapping is required.

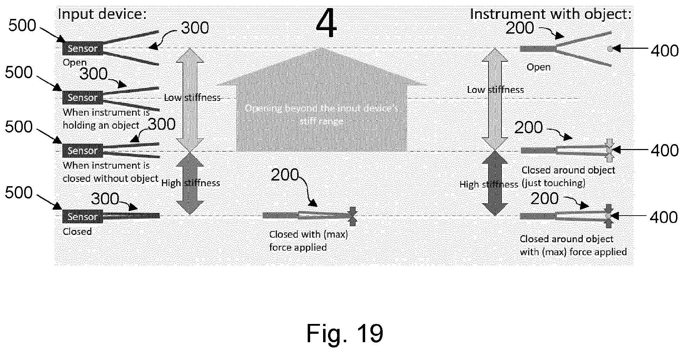

**Fig. 19** shows how operation ranges for the input device 300 are dynamically re-mapped upon releasing the object 400.

When the object 400 is released, i.e., the gripping force on the object 400 becomes zero, operation ranges for the input device 300 shall be re-mapped accordingly in order to prevent mismatch-related errors.

Advantageously, in the present embodiment, the controller is configured to:
actuate the end-effector to transition the surgical instrument 200 from the first state to the second state upon detecting that a force exerted by the user on the user interface of the input device 300 is above a threshold value corresponding to the transition point, and
actuate the end-effector to transition the surgical instrument 200 from the second state to the first state upon detecting that a force exerted by the user on the user interface of the input device 300 is below the threshold value corresponding to the transition point.

That is, until the exerted force is not above or falls below the predefined threshold value, transition from the first state to the second state, or vice versa, does not occur.

For instance, when a force exerted by the user on the user interface is decreased while an object 400 is grasped between the first and second arms 202, 204 of the instrument 200, the object 400 is not completely released until said force falls below a predefined threshold value.

This is advantageous in case is it required to slightly release the gripping force, e.g., for object 400 repositioning, but without determining complete releasing of the object 400.

In one configuration, the end-effector comprises an end-effector base.

The end-effector further comprises first and second movable fingers, distally extending from the end-effector base.

The first and second movable fingers capable of implementing an opening/closing movement with respect to each other.

In detail, the opening/closing movement of the first and second fingers can be obtained by the provision of a hinge located between the fingers and a base portion of the instrument, allowing for movement of both fingers with the same, inverted magnitude.

Advantageously, the end-effector may further comprise a surgical instrument adapter, e.g., of the kind shown in **Fig. 3** and described in WO2022233585A1.

The surgical instrument adapter is configured to transmit a movement of a tip portion of the first and second movable fingers of the end-effector to the surgical instrument.

A sterile drape can be provided, covering the end-effector.

This allows maintaining the sterile barrier.

The invention further provides a method of mapping a user input device 300 in an end-effector system ad described in the foregoing.

In the present embodiment, the method at least comprises:
defining a first state of the surgical instrument 200, characterized by a first value of at least one predefined parameter, said first state corresponding to an open state of the instrument;
defining a second state of the surgical instrument 200, characterized by a second value of said at least one predefined parameter, different from the first value, said second state corresponding to a closed state of the instrument 200;
defining an original transition point between the first state and the second state of the surgical instrument 200, based on a detected change in said at least one predefined parameter from the first value to the second value, and vice versa, and
mapping operation ranges for the input device 300 based on the defined original transition point.

The second state can be a state where tip portions of first and second arms 202, 204 of the surgical instrument 200 contact each other.

Alternatively, the second state can be a state where the first and second arms 202, 204 of the surgical instrument 200 hold an object 400 (e.g., a surgical needle or suture) therebetween.

In this case, the method of the present embodiment further comprises:
when the object 400 is grasped between the first and second arms 202, 204 of the surgical instrument 200, determining that the transition point between the first state and the second state of the surgical instrument 200 has changed;
defining an updated transition point between the first state and the second state of the surgical instrument 200, and
re-mapping operation ranges for the input device 300 based on the defined updated transition point.

When the object 400 is released, operation ranges for the input device 300 shall be re-mapped to the original state, i.e., based on the original transition point of the surgical instrument 200.

Accordingly, in the present embodiment, the method further comprises:
when the object 400 is released, determining that the transition point between the first state and the second state of the surgical instrument 200 is back to the original transition point, and
re-mapping operation ranges for the input device 300 based on the original transition point between the first state and the second state.

The method can be implemented through a computer-readable non-volatile storage medium comprising computer-readable instructions that, when executed by a processing means, causes said processing means to implement and/or process and/or perform the method steps described above.

In connection with the above disclosure, the following aspects are explicitly disclosed:
**Aspect 1:** An end-effector system for a surgical, microsurgical or super-microsurgical robot arm, the system at least comprising:
   a hinged surgical instrument (200) configured to switch between an open state and a closed state, said surgical instrument (200) comprising a first arm (202) and a second arm (204), connected through a hinge;
   an end-effector, configured to control operation of the surgical instrument (200);
   a user input device (300) comprising a user interface configured to receive an input from a user, and
   a controller,
wherein the controller is configured at least to:
   define a first state of the surgical instrument (200), characterized by a first value of at least one predefined parameter, said first state corresponding to an open state of the instrument (200);
   define a second state of the surgical instrument (200), characterized by a second value of said at least one predefined parameter, different from the first value, said second state corresponding to a closed state of the instrument (200);
   define an original transition point between the first state and the second state of the surgical instrument (200), based on a detected change in said at least one predefined parameter from the first value to the second value, and vice versa, and
   map operation ranges for the input device (200), based on the defined original transition point.
**Aspect 2:** The system of Aspect 1, wherein the second state is a state where tip portions of the first and second arms (202, 204) of the surgical instrument (200) contact each other.
**Aspect 3:** The system of Aspect 1, wherein the second state is a state where the first and second arms (202, 204) of the surgical instrument (200) hold an object (400) therebetween,
   wherein the controller is further configured to:
   when the object (400) is grasped between the first and second arms (202, 204) of the surgical instrument (200), determine that the transition point between the first state and the second state of the surgical instrument (200) has changed;
   define an updated transition point between the first state and the second state of the surgical instrument (200), and
   re-map operation ranges for the input device (300) based on the defined updated transition point.
**Aspect 4:** The system of Aspect 3, wherein the controller is further configured to:
   when the object (400) is released, determine that the transition point between the first state and the second state of the surgical instrument (200) is back to the original transition point, and
   re-map operation ranges for the input device (400) based on the original transition point.
**Aspect 5:** The system of Aspects 3 or 4, wherein said object (400) is one of:
   a surgical needle;
   a suture wire, or
   a living tissue of a patient, in particular a blood vessel.
**Aspect 6:** The system of any of the preceding Aspects, wherein the controller is further configured to:
   actuate the end-effector to transition the surgical instrument (200) from the first state to the second state upon detecting that a force exerted by the user on the user interface of the input device (300) is above a threshold value corresponding to the transition point, and
   actuate the end-effector to transition the surgical instrument (200) from the second state to the first state upon detecting that a force exerted by the user on the user interface of the input device (300) is below the threshold value corresponding to the transition point.
**Aspect 7:** The system of any of the preceding Aspects, wherein the input device (300) has at least a first stiffness condition characterized by a first stiffness value, and a second stiffness condition characterized by a second stiffness value, higher than the first stiffness value.
**Aspect 8:** The system of Aspect 7, wherein the transition point between the first stiffness condition and the second stiffness condition of the input device (300) corresponds to the transition point between the first state and the second state of the surgical instrument (200).
**Aspect 9:** The system of Aspect 7, wherein:
   the transition point between the first stiffness condition and the second stiffness condition of the input device (300) is offset with respect to the transition point between the first state and the second state of the surgical instrument (200),
   preferably wherein the transition point between the first stiffness condition and
   the second stiffness condition of the input device (300) is prior in time with respect to the transition point between the first state and the second state of the surgical instrument (200).
**Aspect 10:** The system of any of Aspects 7 to 9, wherein:
   the first stiffness condition of the input device (300) is determined by a first elastic means (302), actuated over an entire opening/closing range of the surgical instrument (200), and
   the second stiffness condition of the input device (300) is determined by a second elastic means (304), configured to provide additional stiffness upon being operated,
   preferably wherein said first and second elastic means (302, 304) is a spring.
**Aspect 11:** The system of Aspect 7, wherein:
   the input device (300) further has at least one intermediate stiffness condition between the first stiffness condition and the second stiffness condition,
   characterized by an intermediate stiffness value,
   preferably wherein said intermediate stiffness condition of the input device (300) is determined by an additional elastic means (306),
   more preferably wherein said additional elastic (306) means is a spring.
**Aspect 12:** The system of any of the preceding Aspects, wherein:
   the system further comprises a sensor means (500) configured to detect a change for said at least one predefined parameter from the first value to the second value, and vice versa,
   preferably wherein said at least one predefined parameter includes one or more among stiffness, coordinate points for the instrument tip, and a position and/or force at the instrument tip.
**Aspect 13:** The system of any of the preceding Aspects, wherein the input device (300) is in the shape of a joystick.
**Aspect 14:** The system of any of the preceding Aspects, wherein the end-effector comprises:
   an end-effector base,
   a first movable finger, and
   a second movable finger,
   wherein said first and second movable fingers distally extend from the end-effector base.
**Aspect 15:** The system of Aspect 14, wherein the end-effector further comprises a surgical instrument adapter, configured to transmit a movement of a tip portion of the first and second movable fingers of the end-effector to the surgical instrument.
**Aspect 16:** The system of any of the preceding Aspects, wherein the system further comprises a sterile drape configured to cover the end-effector.
**Aspect 17:** A surgical, microsurgical or super-microsurgical robot arm comprising an end-effector system as defined in any of Aspects 1 to 16.
**Aspect 18:** A method of mapping a user input device (400) in an end-effector system as defined in any of Aspects 1 to 16, the method at least comprising:
   defining a first state of the surgical instrument (200), characterized by a first value of at least one predefined parameter, said first state corresponding to an open state of the instrument (200);
   defining a second state of the surgical instrument (200), characterized by a second value of said at least one predefined parameter, different from the first value, said second state corresponding to a closed state of the instrument (200);
   defining an original transition point between the first state and the second state of the surgical instrument (200), based on a detected change in said at least one predefined parameter from the first value to the second value, and vice versa, and
   mapping operation ranges for the input device (300) based on the defined original transition point.
**Aspect 19:** The method of Aspect 18, wherein the second state is a state where tip portions of the first and second arms (202, 204) of the surgical instrument (200) contact each other.
**Aspect 20:** The method of Aspect 18, wherein the second state is a state where the first and second arms (202, 204) of the surgical instrument (200) hold an object (400) therebetween, the method further comprising:
   when the object (400) is grasped between the first and second arms (202, 204) of the surgical instrument (200), determining that the transition point between the first state and the second state of the surgical instrument (200) has changed;
   defining an updated transition point between the first state and the second state of the surgical instrument (200), and
   re-mapping operation ranges for the input device (300) based on the defined updated transition point.
**Aspect 21:** The system of Aspect 20, wherein the method further comprises:
   when the object (400) is released, determining that the transition point between the first state and the second state of the surgical instrument (200) is back to the original transition point, and
   re-mapping operation ranges for the input device (300) based on the original transition point between the first state and the second state.
**Aspect 22:** A computer-readable non-volatile storage medium comprising computer-readable instructions that, when executed by a processing means, causes said processing means to implement and/or process and/or perform the method steps defined in Aspects 18 to 21.

### Bibliography

**[1]** El Rassi I, El Rassi JM. A review of haptic feedback in tele-operated robotic surgery. J Med Eng Technol. 2020 Jul;44(5):247-254. doi: 10.1080/03091902.2020.1772391. Epub 2020 Jun 23. PMID: 32573288.
**[2]** Bergholz M, Ferle M, Weber BM. The benefits of haptic feedback in robot assisted surgery and their moderators: a meta-analysis. Sci Rep. 2023 Nov 6;13(1):19215. doi: 10.1038/s41598-023-46641-8. PMID: 37932393; PMCID: PMC10628231.

### Reference list

- 100: Robotic system
- 102: Base station
- 104: Base column
- 106: Display module
- 108: Suspension arm
- 110: Fork-like element
- 112: (First) surgical, microsurgical or super-microsurgical robot arm
- 114: (Second) surgical, microsurgical or super-microsurgical robot arm
- 116: Surgical instrument
- 118: Surgical instrument
- 120: Wheels

- 200: Hinged surgical instrument
- 202: First arm of the surgical instrument
- 204: Second arm of the surgical instrument

- 300: User input device
- 302: First elastic means, first spring
- 304: Second elastic means, second spring
- 306: Additional elastic means, third spring

- 400: Object
- B: Operating bed
- M: Microscope
- S1: First surgeon
- S2: Second surgeon
- P: Patient
- OR: Operating room

## Claims

1. An end-effector system for a surgical, microsurgical or super-microsurgical robot arm, the system at least comprising:
a hinged surgical instrument (200) configured to switch between an open state and a closed state, said surgical instrument (200) comprising a first arm (202) and a second arm (204), connected through a hinge;
an end-effector, configured to control operation of the surgical instrument (200);
a user input device (300) comprising a user interface configured to receive an input from a user, and
a controller,
wherein the controller is configured at least to:
define a first state of the surgical instrument (200), **characterized by** a first value of at least one predefined parameter, said first state corresponding to an open state of the instrument (200);
define a second state of the surgical instrument (200), **characterized by** a second value of said at least one predefined parameter, different from the first value, said second state corresponding to a closed state of the instrument (200);
define an original transition point between the first state and the second state of the surgical instrument (200), based on a detected change in said at least one predefined parameter from the first value to the second value, and vice versa, and
map operation ranges for the input device (200), based on the defined original transition point.

2. The system of claim 1,
**characterized in that**
the second state is a state where tip portions of the first and second arms (202, 204) of the surgical instrument (200) contact each other.

3. The system of claim 1,
**characterized in that**
the second state is a state where the first and second arms (202, 204) of the surgical instrument (200) hold an object (400) therebetween,
preferably wherein said object is one of a surgical needle, a suture wire, or a living tissue of a patient, in particular a blood vessel,
wherein the controller is further configured to:
when the object (400) is grasped between the first and second arms (202, 204) of the surgical instrument (200), determine that the transition point between the first state and the second state of the surgical instrument (200) has changed;
define an updated transition point between the first state and the second state of the surgical instrument (200), and
re-map operation ranges for the input device (300) based on the defined updated transition point.

4. The system of claim 3,
**characterized in that**
the controller is further configured to:
when the object (400) is released, determine that the transition point between the first state and the second state of the surgical instrument (200) is back to the original transition point, and
re-map operation ranges for the input device (400) based on the original transition point.

5. The system of any of the preceding claims,
**characterized in that**
the controller is further configured to:
actuate the end-effector to transition the surgical instrument (200) from the first state to the second state upon detecting that a force exerted by the user on the user interface of the input device (300) is above a threshold value corresponding to the transition point, and
actuate the end-effector to transition the surgical instrument (200) from the second state to the first state upon detecting that a force exerted by the user on the user interface of the input device (300) is below the threshold value corresponding to the transition point.

6. The system of any of the preceding claims,
**characterized in that**
the input device (300) has at least a first stiffness condition **characterized by** a first stiffness value, and a second stiffness condition **characterized by** a second stiffness value, higher than the first stiffness value.

7. The system of claim 6,
**characterized in that**
the transition point between the first stiffness condition and the second stiffness condition of the input device (300) corresponds to the transition point between the first state and the second state of the surgical instrument (200).

8. The system of claim 6,
**characterized in that**
the transition point between the first stiffness condition and the second stiffness condition of the input device (300) is offset with respect to the transition point between the first state and the second state of the surgical instrument (200),
preferably wherein the transition point between the first stiffness condition and the second stiffness condition of the input device (300) is prior in time with respect to the transition point between the first state and the second state of the surgical instrument (200).

9. The system of any of claims 6 to 8,
**characterized in that**
the first stiffness condition of the input device (300) is determined by a first elastic means (302), actuated over an entire opening/closing range of the surgical instrument (200), and
the second stiffness condition of the input device (300) is determined by a second elastic means (304), configured to provide additional stiffness upon being operated,
preferably wherein said first and second elastic means (302, 304) is a spring.

10. The system of claim 6,
**characterized in that**
the input device (300) further has at least one intermediate stiffness condition between the first stiffness condition and the second stiffness condition, **characterized by** an intermediate stiffness value,
preferably wherein said intermediate stiffness condition of the input device (300) is determined by an additional elastic means (306),
more preferably wherein said additional elastic (306) means is a spring.

11. The system of any of the preceding claims,
**characterized in that**
the system further comprises a sensor means (500) configured to detect a change for said at least one predefined parameter from the first value to the second value, and vice versa,
preferably wherein said at least one predefined parameter includes one or more among stiffness, coordinate points for the instrument tip, and a position and/or force at the instrument tip.

12. A method of mapping a user input device (400) in an end-effector system as defined in any of claims 1 to 11, the method at least comprising:
defining a first state of the surgical instrument (200), **characterized by** a first value of at least one predefined parameter, said first state corresponding to an open state of the instrument (200);
defining a second state of the surgical instrument (200), **characterized by** a second value of said at least one predefined parameter, different from the first value, said second state corresponding to a closed state of the instrument (200);
defining an original transition point between the first state and the second state of the surgical instrument (200), based on a detected change in said at least one predefined parameter from the first value to the second value, and vice versa, and
mapping operation ranges for the input device (300) based on the defined original transition point.

13. The method of claim 12,
**characterized in that**
the second state is a state where tip portions of the first and second arms (202, 204) of the surgical instrument (200) contact each other.

14. The method of claim 12,
**characterized in that**
the second state is a state where the first and second arms (202, 204) of the surgical instrument (200) hold an object (400) therebetween,
wherein the method further comprises:
when the object (400) is grasped between the first and second arms (202, 204) of the surgical instrument (200), determining that the transition point between the first state and the second state of the surgical instrument (200) has changed;
defining an updated transition point between the first state and the second state of the surgical instrument (200), and
re-mapping operation ranges for the input device (300) based on the defined updated transition point.

15. The method of claim 14,
**characterized in that**
the method further comprises:
when the object (400) is released, determining that the transition point between the first state and the second state of the surgical instrument (200) is back to the original transition point, and
re-mapping operation ranges for the input device (300) based on the original transition point between the first state and the second state.
